# EUROPÄISCHE PATENTSCHRIFT

(11) **EP 4 362 736 B1**
(45) Veröffentlichungstag und Bekanntmachung des Hinweises auf die Patenterteilung: **14.01.2026**
(21) Anmeldenummer: 22740373.0
(22) Anmeldetag: 29.06.2022
(51) Int. Cl.: A42B 3/22, A42B 3/32

(54) **SCHUTZHELM**
PROTECTIVE HELMET
CASQUE DE PROTECTION

(30) Priorität: 29.06.2021 DE 102021116755; 05.07.2021 DE 102021117299
(43) Veröffentlichungstag der Anmeldung: 08.05.2024
(73) Patentinhaber: Schuberth GmbH, 39126 Magdeburg (DE)
(72) Erfinder: REITEBUCH, Sebastian, 10715 Berlin (DE)
(74) Vertreter: Patentanwälte Bauer Vorberg Kayser
(86) Internationale Anmeldenummer: PCT/EP2022/067881
(87) Internationale Veröffentlichungsnummer: WO 2023/275136

(56) Entgegenhaltungen:
- EP-A1- 0 797 935
- EP-A1- 1 265 506
- US-A1- 2013 081 199

## Beschreibung

Die Erfindung betrifft einen Schutzhelm und insbesondere einen Motorradschutzhelm.

Vor allem als Klapphelme bezeichnete Schutzhelme weisen sowohl ein Kinnteil als auch ein Visier auf, welche beide jeweils verschwenkt werden können. Ist das Kinnteil in der unteren Position, deckt es die Kinnpartie des Schutzhelmträgers ab. In dieser Lage des Kinnteils kann das Visier einerseits in die geschlossene Position gebracht werden, in der der Schutzhelm dann insgesamt vollständig geschlossen ist, und andererseits in die geöffnete Position gebracht werden. In dieser geöffneten Position gibt das Visier die Sichtöffnung frei und ist auf eine Lage oberhalb der Sichtöffnung verschwenkt. Auf diese Weise kann der Fahrer den Helminnenbereich lüften und mehr Frischluft erhalten.

Auch das Kinnteil kann regelmäßig in eine obere Position verschwenkt werden. Ist vor einem solchen Verschwenken das Visier in der geschlossenen Position, dann wird das Visier mit dem Kinnteil nach oben verschwenkt und behält so seine Relativlage zu dem Kinnteil. Verschwenkt der Helmträger des Kinnteil aus der oberen Position wieder zurück in die untere Position, so folgt das Visier mit dem Kinnteil und befindet sich ebenfalls wieder in derselben Position wie zu Beginn. Ist hingegen das Kinnteil in der unteren Position und das Visier in der geöffneten Position und wird aus einer solchen Lage heraus das Kinnteil dann in die obere Position verschwenkt, so verbleibt das Visier entweder in seiner Position relativ zur Helmschale, sodass sich also das Kinnteil auf das Visier zubewegt, oder das Visier bewegt sich allenfalls in geringerem Maße als das Kinnteil weiter in Richtung der geöffneten Position. Im Ergebnis bewegen sich Kinnteil und Visier aufeinander zu. Hat das Kinnteil die obere Position erreicht, so entsprechen die Lage des Kinnteils und des Visiers derjenigen, die sie auch nach einem Verschwenken des Kinnteils nach oben bei geschlossenem Visier hätten. Der Grund hierfür ist, dass das Visier insbesondere aus Gründen der Aerodynamik nicht beliebig weit nach oben bewegt werden soll. Da die Schwenkmechanik des Visiers diese relativ zu dem Kinnteil fixiert, führt ein Zurückschwenken des Kinnteils in die untere Position nun dazu, dass das Visier dem Kinnteil folgt und sich dann in der geschlossenen Position befindet. Nachteilig daran ist, dass dies nicht dem Zustand des Visiers vor dem Verschwenken des Kinnteils aus der unteren Position heraus entspricht. Das Verschwenken und wieder Zurückschwenken des Kinnteils führt also zu einem Verschließen des Visiers.

EP 0 797 935 offenbart ein Schutzhelm mit einer Helmschale, einem Kinnteil, einer Kopplungsmechanik und einem Visier.

Ausgehend von diesem Stand der Technik besteht die Aufgabe der Erfindung daher darin, den bekannten Schutzhelm so weiterzuentwickeln und zu verbessern, dass er bezüglich der Positionierung des Visiers und des Kinnteils bei Schwenkbewegungen einen verbesserten Komfort bietet.

Diese Aufgabe wird durch einen Schutzhelm mit den Merkmalen des kennzeichnenden Teils von Anspruch 1 gelöst.

Wesentlich für die Erfindung ist die Erkenntnis, dass eine Kopplungsmechanik vorgesehen werden kann, welche sich die Lage des Visiers zum Kinnteil vor dem Verschwenken des Kinnteils "merkt". Auch wenn sich die relative Lage des Visiers zum Kinnteil in der oberen Position des Kinnteils vorübergehend verändert, so kann nach dem Zurückschwenken des Kinnteils in die untere Position das Visier in seiner früheren Position verbleiben bzw. in diese zurückkehren. Auf diese Weise weist der Schutzhelm eine Memory-Funktion bezüglich der Lage des Visiers bei Schwenkbewegungen des Kinnteils auf.

Der vorschlagsgemäße Schutzhelm, bei dem es sich insbesondere um einen Motorradschutzhelm handeln kann, weist eine Helmschale, ein Kinnteil zum Abdecken einer Kinnpartie eines Schutzhelmträgers, eine Kopplungsmechanik, durch welche das Kinnteil zwischen einer unteren Position und einer oberen Position verschwenkbar an der Helmschale befestigt ist und ein Visier auf, welches Visier beim Kinnteil in der unteren Position durch die Kopplungsmechanik eine jeweilige Schwenkposition zwischen einer geschlossenen Position zur zumindest teilweisen Abdeckung eines Sichtbereichs des Schutzhelmträgers und einer geöffneten Position zum Freigeben des Sichtbereichs einnehmen kann.

Die Helmschale kann insbesondere eine Außenschale zur Verteilung von Aufprallkräften sowie eine von der Außenschale aufgenommene Innenschicht zur Dämpfung von Aufprallkräften aufweisen. Neben der Außenschale und der Innenschicht werden hier und nachstehend vorzugsweise auch alle anderen starr mit der Helmschale verbundenen Komponenten des Schutzhelms unter den Begriff der Helmschale gefasst. Neben dem Visier kann der Schutzhelm auch einen ggf. ebenfalls verschwenkbaren Sonnenschutz oder eine sonstige Vorrichtung zum Schutz einer Augenpartie des Schutzhelmträgers aufweisen. Insbesondere kann es sich bei dem Schutzhelm um einen Klapphelm handeln. Es ist bevorzugt, dass der Schutzhelm bei dem Kinnteil in der unteren Position und dem Visier in der geschlossenen Position im Wesentlichen vollständig geschlossen ist. Vorzugsweise deckt das Visier in der geschlossenen Position den Sichtbereich des Schutzhelmträgers vollständig ab.

Es kann sein, dass das Kinnteil neben der unteren Position und der oberen Position im Grunde beliebig viele Zwischenpositionen einnehmen kann. Ebenfalls kann es sein, dass das Visier zwischen der geschlossenen Position und der geöffneten Position im Grunde beliebig viele Zwischenpositionen einnehmen kann.

Der vorschlagsgemäße Schutzhelm ist dadurch gekennzeichnet, dass die Kopplungsmechanik dazu eingerichtet ist, auf ein Verschwenken des Kinnteils aus der oberen Position in die untere Position nach einem vorherigen Verschwenken des Kinnteils aus der unteren Position in die obere Position zu bewirken, dass das Visier eine von der geschlossenen Position abweichende Zuvor-Schwenkposition innehat, welche Zuvor-Schwenkposition das Visier vor dem Verschwenken des Kinnteils aus der unteren Position in die obere Position innehatte. Bei dieser Zuvor-Schwenkposition kann es sich um eine im Grunde beliebige Schwenkposition des Visiers handeln mit Ausnahme der geschlossenen Position.

Bei Schutzhelmen aus dem Stand der Technik ist es wie bereits beschrieben regelmäßig der Fall, dass wenn das Visier in der geschlossenen Position war beim Kinnteil in der unteren Position, dann das Visier auch wieder in der geschlossenen Position ist nachdem das Kinnteil in die obere Position und dann wieder in die untere Position zurück verschwenkt wird. Das liegt daran, dass durch das Verschwenken des Kinnteils in die obere Position und wieder zurück Verschwenken in die untere Position das Visier regelmäßig in die geschlossene Position (bezogen auf das Kinnteil) überführt wird, und zwar unabhängig von der vorherigen Schwenkposition des Visiers. Bei dem vorschlagsgemäßen Schutzhelm gilt das also für Schwenkpositionen des Visiers außer der geschlossenen Position. Es bleibt aber auch beim vorschlagsgemäßen Schutzhelm unbenommen, dass diese Rückkehr in die vorherige Position des Visiers zusätzlich auch für die geschlossene Position des Visiers gilt. Diese Memory-Funktion auch für die geschlossene Position des Visiers ist also keinesfalls ausgeschlossen. Es ist bloß nicht ausreichend, eine solche Memory-Funktion nur für die geschlossene Position des Visiers vorzusehen.

Anders ausgedrückt führt ein Verschwenken des Kinnteils von der unteren in die obere Position und wieder zurück dazu, dass das Visier entweder in derjenigen Position verbleibt, welche es dem vor dem Verschwenken des Kinnteils in die obere Position hatte - also die Zuvor-Schwenkposition - oder in diese frühere Position zurückbewegt wird. Anders ausgedrückt kann das Visier zwischenzeitlich seine Position bezüglich des Kinnteils wechseln, kehrt dann aber wieder in seine frühere Position zurück.

Eine bevorzugte Ausführungsform des Schutzhelms ist dadurch gekennzeichnet, dass die Kopplungsmechanik dazu eingerichtet ist, auf ein Verschwenken des Kinnteils aus der unteren Position in die obere Position bei dem Visier in der geöffneten Position das Kinnteil derart zum Visier zu bewegen, dass das Visier in der oberen Position des Kinnteils näher am Kinnteil ist. Dies kann insbesondere dadurch erfolgen, dass das Visier entweder überhaupt nicht oder nur in geringerem Maße der Schwenkbewegung des Kinnteils folgt.

Eine weitere bevorzugte Ausführungsform des Schutzhelms ist dadurch gekennzeichnet, dass auf ein Verschwenken des Kinnteils aus der unteren Position in die obere Position bei einem Visier in der geschlossenen Position das Kinnteil seine relative Lage zum Visier behält. Wenn das Visier also vor dem Verschwenken mit dem Kinnteil in Eingriff steht oder die Vorderseite des Schutzhelms verschließt, dann gilt das auch nach dem Verschwenken des Kinnteils. Bevorzugt ist, dass auf ein Verschwenken des Kinnteils aus der unteren Position in die obere Position bei einem Visier in der geschlossenen Position das Visier durch das Kinnteil und alternativ oder zusätzlich durch die Kopplungsmechanik mitgenommen wird.

Gemäß einer bevorzugten Ausführungsform des Schutzhelms ist vorgesehen, dass die Kopplungsmechanik eine Rastvorrichtung aufweist, welche schwenkbar mit der Helmschale gekoppelt ist. Anders ausgedrückt besteht zwar eine direkte oder indirekte mechanische Verbindung zwischen der Rastvorrichtung und der Helmschale, die Rastvorrichtung lässt sich jedoch grundsätzlich gegenüber der Helmschale verschwenken. Bevorzugt ist weiter, dass das Visier zum Verändern der Schwenkposition mit der Rastvorrichtung schwenkbar gekoppelt ist. Auch diese Kopplung zwischen dem Visier und der Rastvorrichtung, welche Kopplung die Veränderung der Schwenkposition ermöglicht, kann direkt oder indirekt sein. Bevorzugt ist weiter, dass die Schwenkposition des Visiers durch eine relative Verschwenkung des Visiers zur Rastvorrichtung und eine relative Verschwenkung der Rastvorrichtung zur Helmschale definiert wird. Dadurch wird also zumindest für einige Schwenkpositionen des Visiers keine Lage der Rastvorrichtung gegenüber der Helmschale eindeutig definiert. Vielmehr hat die Rastvorrichtung einen entsprechenden Freiheitsgrad hinsichtlich ihrer Schwenkposition. Es kann weiter sein, dass das Visier mit der Rastvorrichtung eine jeweilige Rastverbindung in einer Vielzahl von relativen Lagen zum Rastvorrichtung eingehen kann. Vorzugsweise wird das Visier dann durch die Rastverbindung in der jeweiligen relativen Lage gehalten.

Gemäß einer weiteren bevorzugten Ausführungsform des vorschlagsgemäßen Schutzhelms ist vorgesehen, dass die Rastvorrichtung gegenüber dem Kinnteil schwenkbar gelagert ist. Insbesondere kann es sein, dass die Rastvorrichtung dazu eingerichtet ist, bei einem Schwenken der Rastvorrichtung bezogen auf die Helmschale das Visier mitzunehmen, und zwar vorzugsweise durch die jeweilige Rastverbindung. Dies ermöglicht es, ein Verschwenken des Visiers durch Betätigung der Rastvorrichtung zu bewirken. Eine solche Betätigung der Rastvorrichtung erfolgt regelmäßig allerdings nicht durch direkten manuellen Eingriff des Schutzhelmträgers, sondern durch den Eingriff einer anderen Komponente des Schutzhelms. Diese andere Komponente kann dann ihrerseits direkt oder indirekt durch den Schutzhelmträger betätigt werden.

Dieser zusätzliche Freiheitsgrad der Rastvorrichtung kann auch wahlweise wieder aufgehoben werden. Auf diese Art kann die beschriebene Memoryfunktion jedenfalls zeitweilig abgestellt werden. Eine bevorzugte Ausführungsform des vorschlagsgemäßen Schutzhelms ist dadurch gekennzeichnet, dass die Kopplungsmechanik eine lösbare Arretierungsvorrichtung zum starren Koppeln der Rastvorrichtung mit dem Kinnteil aufweist. Ist die Rastvorrichtung mit dem Kinnteil starr gekoppelt, dann führt eine geschlossene Position des Visiers in der oberen Position des Kinnteils auch zu einer geschlossenen Position des Visiers, wenn das Kinnteil in die untere Position zurück verschwenkt wird.

Grundsätzlich kann die mechanische Interaktion zwischen dem Kinnteil und der Rastvorrichtung beliebig ausgestaltet sein. Gemäß einer bevorzugten Ausführungsform des vorschlagsgemäßen Schutzhelms ist vorgesehen, dass die Kopplungsmechanik einen Rastmitnehmer aufweist, welcher vorzugsweise starr mit dem Kinnteil verbunden ist. Insbesondere kann es sein, dass der Rastmitnehmer in der unteren Position des Kinnteils derart mit der Rastvorrichtung in Eingriff steht, dass die Rastvorrichtung in einer Schließposition der Rastvorrichtung fixiert ist. Damit zwingt das Kinnteil die Rastvorrichtung durch den Rastmitnehmer in eine bestimmte Position - nämlich in die Schließposition - wenn das Kinnteil in der unteren Position ist.

Gemäß einer weiteren bevorzugten Ausführungsform des vorschlagsgemäßen Schutzhelms ist vorgesehen, dass die Kopplungsmechanik dazu eingerichtet ist, auf eine Betätigung des Visiers bei der durch den Rastmitnehmer fixierten Rastvorrichtung zu bewirken, dass das Visier seine Schwenkposition durch eine Veränderung seiner relativen Lage zu der Rastvorrichtung verändert. Anders ausgedrückt steht die Fixierung der Rastvorrichtung einem Verschwenken des Visiers nicht entgegen. Allerdings bewirkt diese Fixierung, dass das Verschwenken des Visiers nicht mit einer entsprechenden Schwenkbewegung der Rastvorrichtung einhergeht, sondern vielmehr eine Relativbewegung zwischen dem Visier und der Rastvorrichtung die Folge ist.

Während also die Rastvorrichtung bevorzugt in einer Position fixiert ist, wenn das Kinnteil in der unteren Position ist, wird eine Beweglichkeit der Rastvorrichtung bevorzugt, wenn das Kinnteil in der oberen Position ist. Dieser Freiheitsgrad erlaubt es der Kopplungsmechanik, sich die vormalige Lage des Visiers entsprechend der relativen Lage von Visier und Rastvorrichtung zu "merken". Entsprechend ist eine bevorzugte Ausführungsform des vorschlagsgemäßen Schutzhelms dadurch gekennzeichnet, dass in der oberen Position des Kinnteils eine Schwenkbewegung der Rastvorrichtung um die Helmschale in mindestens eine Richtung blockierungsfrei ist. Vorzugsweise gilt dies für alle Lagen der Rastvorrichtung. Blockierungsfrei in mindestens eine Richtung bedeutet, dass die Rastvorrichtung in mindestens eine Schwenkrichtung verschwenkt werden kann ohne direkt blockiert zu werden.

Grundsätzlich kann auch die mechanische Interaktion zwischen dem Kinnteil und dem Visier beliebig ausgestaltet sein. Eine weitere bevorzugte Ausführungsform des vorschlagsgemäßen Schutzhelms ist dadurch gekennzeichnet, dass die Kopplungsmechanik einen Visiermitnehmer aufweist, welcher vorzugsweise starr mit dem Kinnteil verbunden ist. Insbesondere kann es sein, dass der Visiermitnehmer mit dem Visier in Eingriff steht, wenn das Kinnteil in der unteren Position und das Visier in der geschlossenen Position ist.

Gemäß einer bevorzugten Ausführungsform des vorschlagsgemäßen Schutzhelms ist vorgesehen, dass auf ein Verschwenken des Kinnteils aus der unteren Position in die obere Position bei dem Visier in der geschlossenen Position das Visier durch den Visiermitnehmer mitgenommen wird. Weiter ist bevorzugt, dass durch das Mitnehmen des Visiers durch den Visiermitnehmer das Visier die Rastvorrichtung mitnimmt. Es folgt dann also nicht nur das Visier der Bewegung des Kinnteils, sondern auch - mittelbar durch das Visier - die Rastvorrichtung der Bewegung des Kinnteils.

Eine weitere bevorzugte Ausführungsform des vorschlagsgemäßen Schutzhelms ist dadurch gekennzeichnet, dass der Visiermitnehmer und/oder der Rastmitnehmer in einem Seitenbereich des Schutzhelms angeordnet ist. Vorzugsweise ist die Kopplungsmechanik beidseitig und im Wesentlichen gegenüberliegend in einem jeweiligen Seitenbereich des Schutzhelms angeordnet.

Gemäß einer weiteren bevorzugten Ausführungsform des vorschlagsgemäßen Schutzhelms ist vorgesehen, dass in der oberen Position des Kinnteils eine Schwenkbewegung des Visiers in Richtung der geschlossenen Position durch den Visiermitnehmer und weiter in Richtung der geöffneten Position durch einen starr mit der Helmschale verbundenen Oberanschlag der Kopplungsmechanik begrenzt wird. Auf diese Weise wird das Visier in der oberen Position des Kinnteils fixiert. Die Lage des Visiers bei der unteren Position des Kinnteils wird dann durch die relative Lage des Visiers zur Rastvorrichtung gespeichert.

Grundsätzlich können die verschiedenen Schwenkbewegungen um jeweils verschiedene Achsen erfolgen. Eine bevorzugte Ausführungsform des vorschlagsgemäßen Schutzhelms ist dadurch gekennzeichnet, dass das Visier und das Kinnteil um eine gemeinsame Schwenkachse verschwenkt werden können. Bevorzugt ist weiter, dass die Rastvorrichtung um die gemeinsame Schwenkachse verschwenkt werden kann.

Eine weitere bevorzugte Ausführungsform des vorschlagsgemäßen Schutzhelms ist dadurch gekennzeichnet, dass der Visiermitnehmer einen Visiervorsprung aufweist, welcher bei einer Schwenkbewegung des Kinnteils eine Schwenkbewegung in einer Schwenkebene auf einem ersten Radius um die Schwenkachse ausführt, welcher erste Radius von einem zweiten Radius um die Schwenkachse verschieden ist, auf welchem zweiten Radius die Rastvorrichtung in der Schwenkebene um die Schwenkachse angeordnet ist. Bei der Schwenkebene handelt es sich um eine Ebene, welche durch die Schwenkachse definiert wird. Indem der Visiervorsprung innerhalb der Schwenkebene radial versetzt zu der Rastvorrichtung angeordnet ist, sind das Kinnteil und die Rastvorrichtung weitgehend frei zueinander positionierbar.

Weitere vorteilhafte und bevorzugte Ausgestaltungen ergeben sich aus der nachfolgenden Beschreibung mit Bezug auf die Figuren. In der lediglich Ausführungsbeispiele wiedergebenden Zeichnung zeigt
- Fig. 1: eine schematische Seitenansicht eines ersten Ausführungsbeispiels eines vorschlagsgemäßen Schutzhelms mit dem Kinnteil in der unteren Position und dem Visier in der geschlossenen Position,
- Fig. 2: eine schematische Seitenansicht des Schutzhelms der Fig. 1 mit dem Kinnteil in der unteren Position und dem Visier in einer Zwischenposition zwischen der geschlossenen Position und der geöffneten Position,
- Fig. 3: eine schematische Seitenansicht des Schutzhelms der Fig. 1 mit dem Kinnteil in der oberen Position und dem Visier relativ zu dem Kinnteil in der geschlossenen Position,
- Fig. 4: eine schematische Draufsicht auf die Kopplungsmechanik des Schutzhelms der Fig. 1 mit dem Kinnteil in der unteren Position und dem Visier in der geschlossenen Position,
- Fig. 5: eine schematische Draufsicht auf die Kopplungsmechanik des Schutzhelms der Fig. 1 mit dem Kinnteil in der unteren Position und dem Visier in der geöffneten Position,
- Fig. 6: eine schematische Draufsicht auf die Kopplungsmechanik des Schutzhelms der Fig. 1 mit dem Kinnteil in der oberen Position und dem Visier in einer kompakten Position,
- Fig. 7: eine schematische Draufsicht auf die Lagerung des Kinnteils an der Helmschale in der Kopplungsmechanik des Schutzhelms der Fig. 1,
- Fig. 8: eine schematische Perspektivansicht auf die Kopplungsmechanik eines zweiten Ausführungsbeispiels eines vorschlagsgemäßen Schutzhelms mit dem Kinnteil und dem Visier in einer jeweiligen Position entsprechend der Fig. 4,
- Fig. 9: eine schematische Perspektivansicht auf die Kopplungsmechanik der Fig. 8 mit dem Kinnteil und dem Visier in einer jeweiligen Position entsprechend der Fig. 5,
- Fig. 10: eine schematische Perspektivansicht auf die Kopplungsmechanik der Fig. 8 mit dem Kinnteil und dem Visier in einer jeweiligen Position entsprechend der Fig. 6 und
- Fig. 11: eine schematische Perspektivansicht auf die Lagerung des Kinnteils an der Helmschale in der Kopplungsmechanik der Fig. 8 in jeweiligen Positionen entsprechend der Fig. 7.

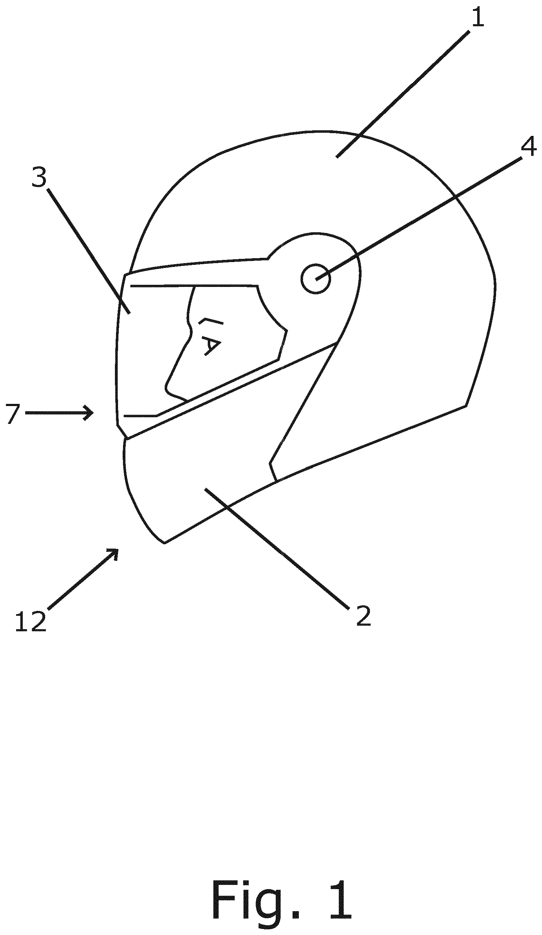

Bei dem in der Fig. 1 dargestellten vorschlagsgemäßen Schutzhelm gemäß dem ersten Ausführungsbeispiel handelt es sich um einen Motorradschutzhelm und speziell um einen Klapphelm. Er weist eine Helmschale 1 sowie ein Kinnteil 2, ein Visier 3 und eine Kopplungsmechanik 4 auf. Das Kinnteil 2 und das Visier 3 können mithilfe der Kopplungsmechanik 4 verschwenkt werden, wobei in den Fig. 1, 2 und 3 jeweils verschiedene Positionen des Kinnteils 2 und des Visiers 3 dargestellt werden.

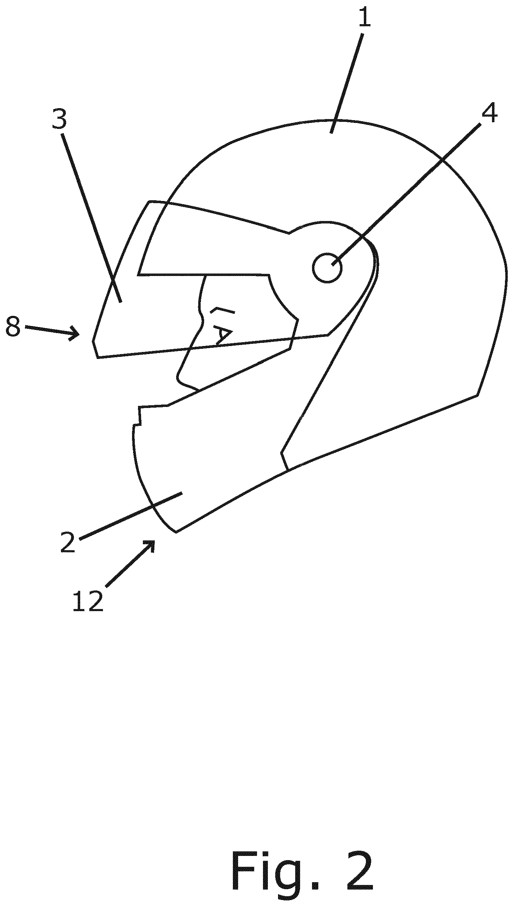

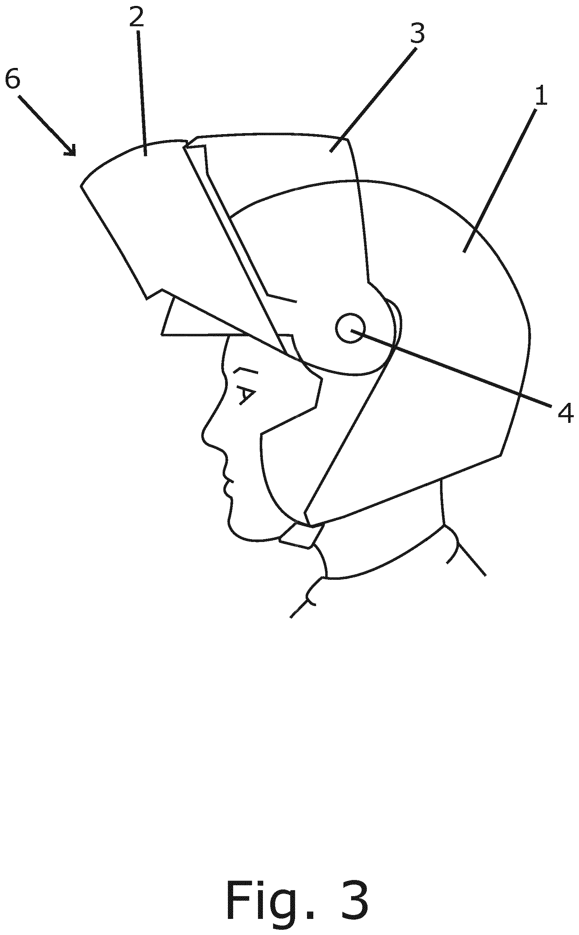

In den Fig. 1 und 2 ist das Kinnteil 2 in seiner unteren Position 12, wohingegen das Kinnteil 2 in der Fig. 3 in seiner oberen Position 6 ist. In der Fig. 1 ist das Visier 3 in seiner geschlossenen Position 7. In der Fig. 2 ist das Visier 3 in einer Zwischenposition zwischen der geschlossenen Position 7 und der geöffneten Position. Diese Zwischenposition des Visiers 3 entspricht hier beispielhaft der Zuvor-Schwenkposition 8. Bei der Schwenkposition des Visiers 3 in der Fig. 3 handelt es sich um eine kompakte Position, welche sogar jenseits der geöffneten Position ist.

Wird nun, ausgehend von der Situation der Fig. 2, das Kinnteil 2 in die obere Position 6 gemäß Fig. 3 verschwenkt, so nimmt das Visier 3 entsprechend die in der Fig. 3 gezeigte kompakte Position ein. Auf ein Zurückschwenken des Kinnteils 2 in die untere Position 12 kehrt auch das Visier 3 in die Zwischenposition gemäß Fig. 3 zurück, speziell also in die Zuvor-Schwenkposition 8. Die Zuvor-Schwenkposition 8 ist also diejenige Schwenkposition, welche das Visier 3 vor dem Verschwenken des Kinnteils 2 innehatte.

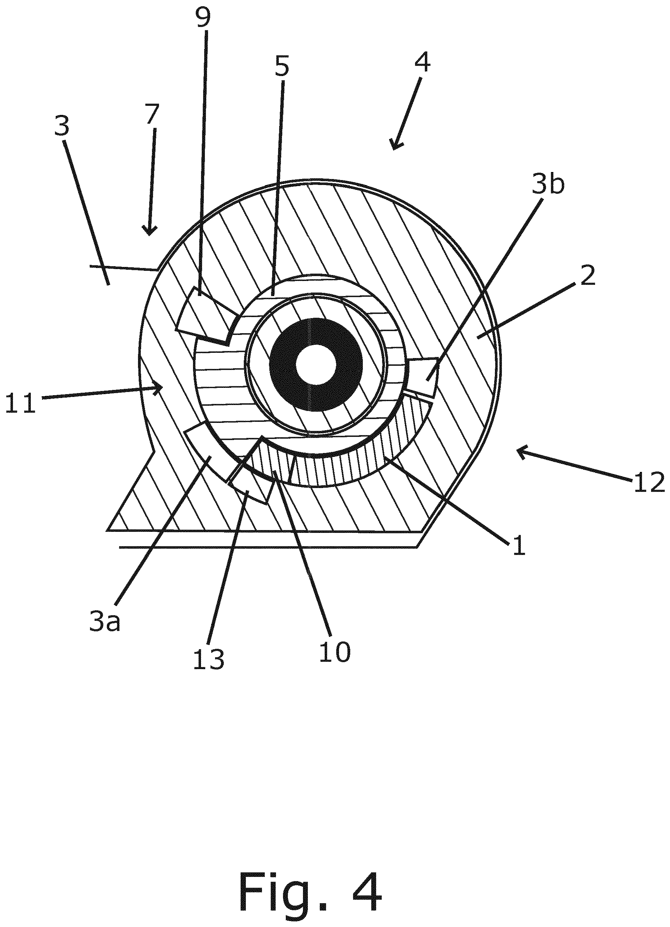

Dieses "Merken" der Zuvor-Schwenkposition 8 und die Rückkehr des Visiers 3 zu ihr wird durch die in den Fig. 4 bis 7 detaillierter dargestellte Kopplungsmechanik 4 ermöglicht.

Die Fig. 4 zeigt die Kopplungsmechanik 4 im Zustand des Schutzhelms gemäß der Fig. 1. In Richtung der Zeichenebene übereinander angeordnet sind - in dieser Reihenfolge - die Helmschale 1, das Kinnteil 2 und das Visier 3. In der Fig. 4 ist durch eine kreisbogenartige Öffnung hindurch lediglich ein starr mit der Helmschale 1 im Übrigen gekoppeltes Bauteil erkennbar, welches gemäß den obigen Feststellungen ebenfalls als Bestandteil der Helmschale 1 angesehen wird. Es sei festgestellt, dass hier schematisch nur ein kleiner Teil dieser Komponenten gezeigt ist und die Verbindung zu den Komponenten im Übrigen nicht dargestellt ist. Für das Visier 3 sind zur Verdeutlichung ein erster Visiervorsprung 3a und ein zweiter Visiervorsprung 3b gesondert dargestellt. Diese sind Bestandteil des Visiers 3 und starr mit dem Visier 3 im Übrigen gekoppelt.

Das Visier 3 kann mit der Rastvorrichtung 5 durch - hier nicht dargestellte Rastpunkte - eine jeweilige Rastverbindung eingehen. Diese Rastpunkte sind so verteilt an der Rastvorrichtung 5 angeordnet, dass in verschiedenen Schwenklagen des Visiers 3 gegenüber der Rastvorrichtung 5 eine solche Rastverbindung hergestellt wird.

Die Kopplungsmechanik 4 weist einen starr mit dem Kinnteil 2 verbundenen Rastmitnehmer 9 auf, welcher in der unteren Position 12 des Kinnteils 2 zusammen mit einem Oberanschlag 10 der Kopplungsmechanik 4, welcher Oberanschlag 10 starr mit der Helmschale 1 gekoppelt ist, die Rastvorrichtung 5 in der gezeigten Lage fixiert. Diese Lage der Rastvorrichtung 5 wird hier als Schließposition 11 bezeichnet.

Die Fixierung der Rastvorrichtung 5 in der Schließposition 11 führt dazu, dass eine Betätigung des Visiers 3 in Richtung der geöffneten Position eine Relativbewegung zwischen dem Visier 3 und der Rastvorrichtung 5 bewirkt. Es löst sich also die bestehende Rastverbindung zwischen dem Visier 3 und der Rastvorrichtung 5 und eine neue Rastverbindung bei einer anderen Lage wird hergestellt.

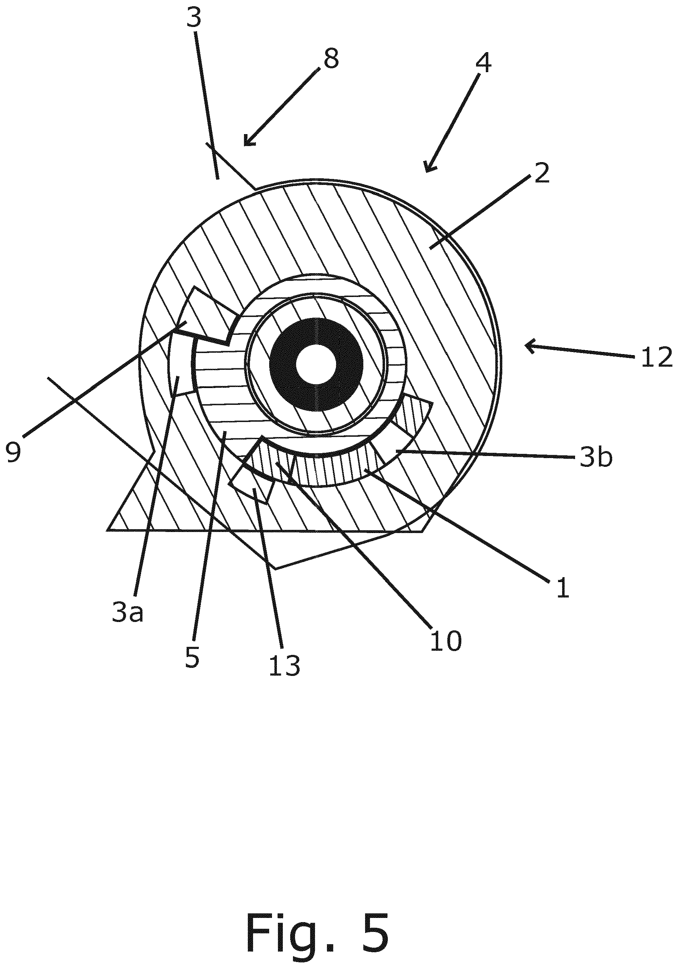

Die beschriebene Betätigung des Visiers 3 führt beispielhaft zu einer Position des Visiers 3 wie in der Fig. 5 gezeigt, welche ebenfalls als Zuvor-Schwenkposition 8 bezeichnet werden kann. Zu beachten ist, dass es sich hier um eine Zuvor-Schwenkposition 8 handelt, welche von der in der Fig. 2 dargestellten Zuvor-Schwenkposition 8 verschieden ist, da das Visier 3 gemäß Fig. 5 weiter geöffnet ist als in der Fig. 2. Bezüglich der Funktionsweise der Kopplungsmechanik 4 ist dieser Unterschied jedoch unwesentlich. Erkennbar hat sich die Position der Rastvorrichtung 5 durch die Betätigung des Visiers 3 nicht verändert.

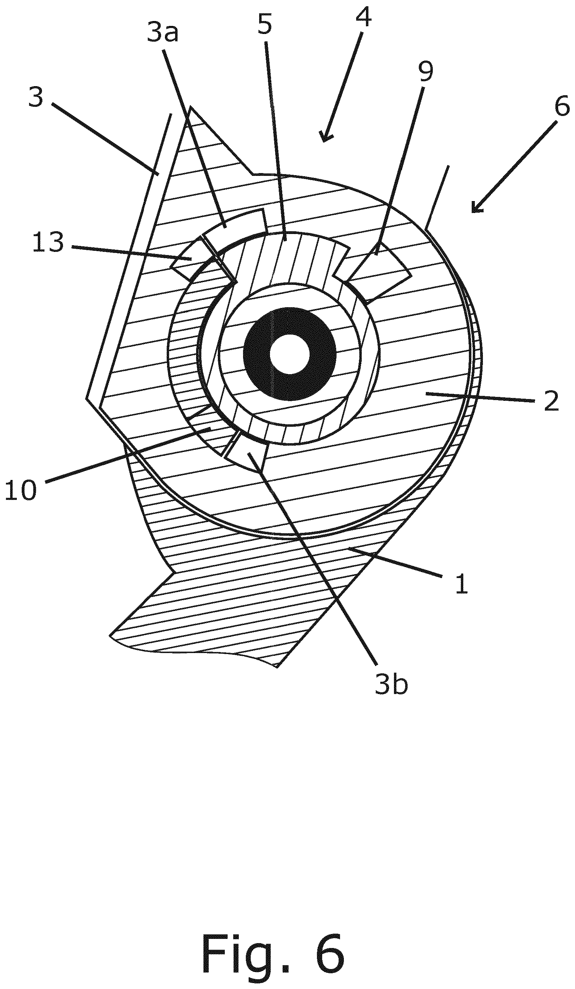

Die Fig. 6 wiederum stellt einen Zustand der Koppelmechanik 4 dar der erreicht wird, wenn ausgehend von dem Zustand der Fig. 4 das Kinnteil 2 aus der unteren Position 12 in die obere Position 6 verschwenkt wird. Diese Schwenkbewegung gibt einerseits die Rastvorrichtung 5 dadurch frei, dass der Rastmitnehmer 9 sich von der Rastvorrichtung 5 löst. Andererseits greift ein ebenfalls starr mit dem Kinnteil 2 gekoppelter und hier als Visiervorsprung ausgebildeter Visiermitnehmer 13 in das Visier 3 und genauer in den ersten Visiervorsprung 3a ein und nimmt auf diese Weise das Visier 3 insgesamt mit. Durch die Rastverbindung des Visiers 3 mit der Rastvorrichtung 5 wird auch die Rastvorrichtung 5 bei dieser Schwenkbewegung mitgenommen, und zwar ohne dass sich die Rastverbindung zwischen Visier 3 und Rastvorrichtung 5 löst. Im Ergebnis tritt eine Lage des Visiers 3 und des Kinnteils 2 wie in der Fig. 3 ein.

Erkennbar ist, dass auf diese Weise die relative Lage zwischen dem Visier 3 und der Rastvorrichtung 5 erhalten bleibt. Gleichzeitig hat die Rastvorrichtung 5 grundsätzlich Spiel bezüglich einer Schwenkbewegung. Speziell greift kein Mitnehmer direkt an der Rastvorrichtung 5 an. Demgegenüber ist das Visier 3 in seiner Lage fixiert, da es in der einen Schwenkrichtung mit dem Visiermitnehmer 13 und in der anderen Schwenkrichtung mit dem Oberanschlag 10 in Eingriff steht. Wird das Kinnteil 2 anschließend wieder in die untere Position 12 zurückgeführt, so stellt sich wieder der Zustand der Fig. 4 ein.

Hieraus lässt sich auch erkennen was passiert, wenn ausgehend von der Situation der Fig. 5 das Kinnteil 2 von der unteren Position 12 in die obere Position 6 verschwenkt wird. Visier 3 und Kinnteil 2 nehmen trotz der gegenüber der Fig. 4 unterschiedlichen Schwenkposition des Visiers 3 vor der Schwenkbewegung - welche Schwenkposition hier wiederum als Zuvor-Schwenkposition 8 zu bezeichnen ist - ebenfalls eine Lage wie in der Fig. 3 ein. Doch trotz identischer Lage des Visiers 3 ist die Konfiguration der Kopplungsmechanik 4 von derjenigen aus der Fig. 6 verschieden, da die relative Lage zwischen Visier 3 und der Rastvorrichtung 5 gemäß der Fig. 5 durch die Rastverbindung beibehalten wird. Bezogen auf die Darstellung der Fig. 6 ist die Rastvorrichtung 5 einfach entgegen dem Uhrzeigersinn verschwenkt, was wiederum durch den vorhandenen Freiheitsgrad für die Rastvorrichtung 5 ermöglicht wird. Das führt dazu, dass bei einem Zurückschwenken des Kinnteils 2 in die untere Position 12 das Visier 3 wieder in seine vorherige Schwenkposition, also in die Zuvor-Schwenkposition 8, zurückkehren kann.

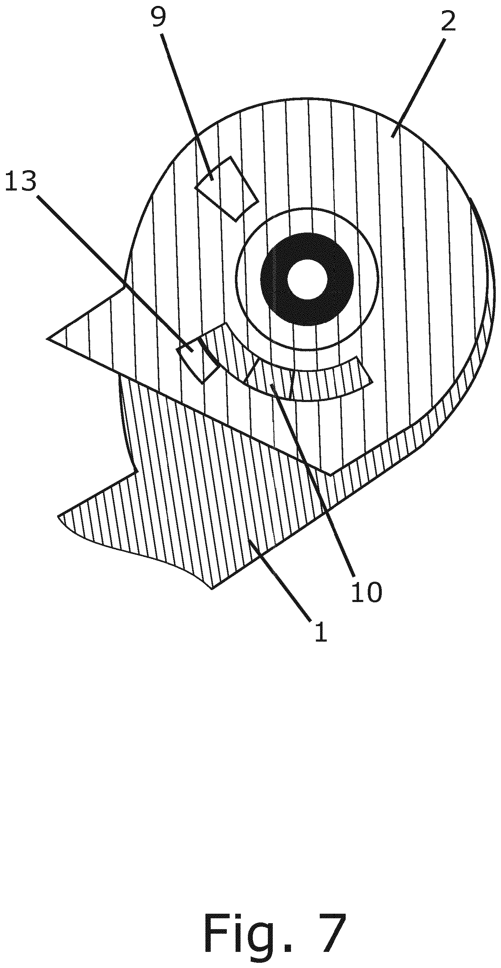

In der Fig. 7 ist zum besseren Verständnis die Kopplungsmechanik 4 ohne Visier 3 und ohne Rastvorrichtung 5 gezeigt.

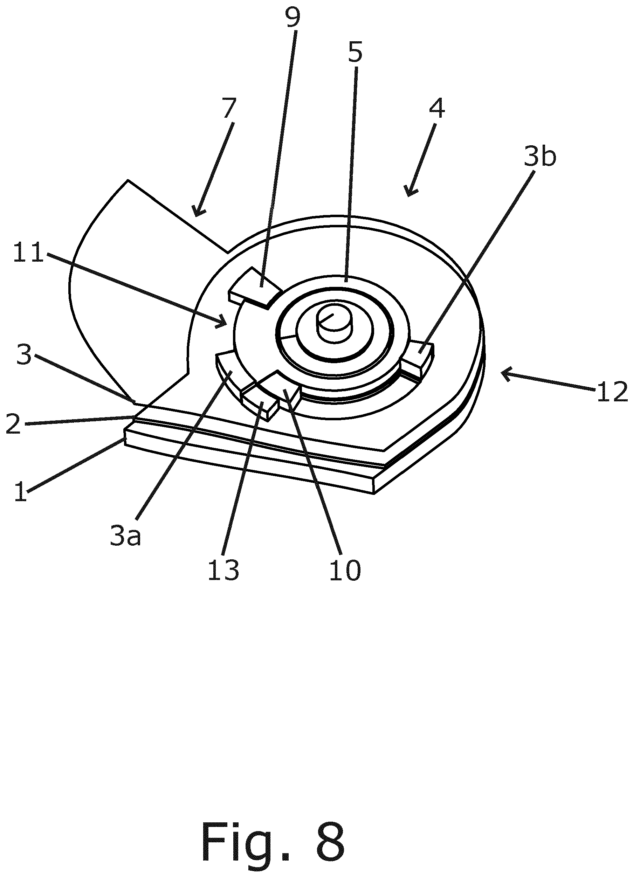

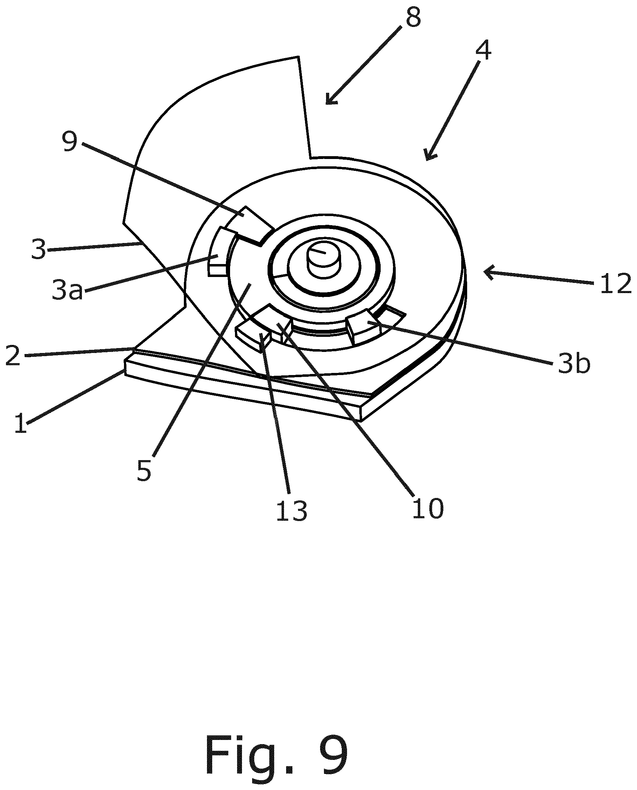

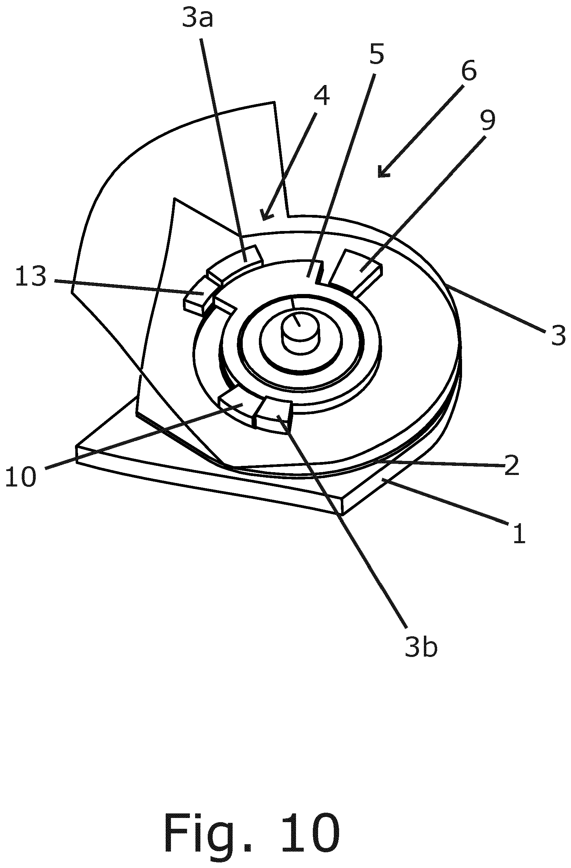

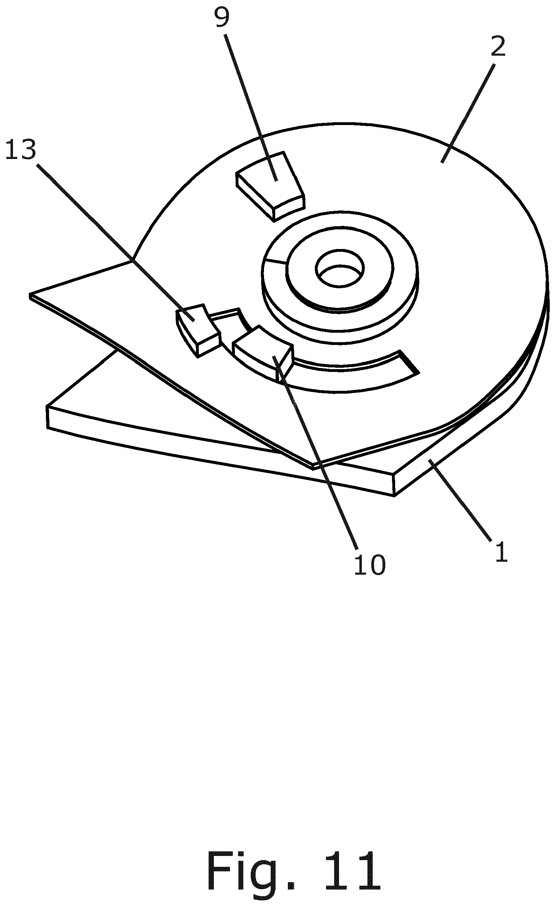

Die Fig. 8 bis 11 zeigen die Kopplungsmechanik 4 bzw. Teile der Kopplungsmechanik 4 eines zweiten Ausführungsbeispiels des vorschlagsgemäßen Schutzhelms in einer schematischen Perspektivansicht.

Die Fig. 8 bis 11 entsprechen dabei in den gezeigten Komponenten sowie in deren prinzipiellen Positionen im Wesentlichen den Fig. 4 bis 7. Unterschiede bestehen lediglich in den speziellen Maßen, Winkeln und Dimensionen und ähnlichen Details, sodass die obigen Feststellungen zu den Fig. 4 bis 7 und zu den darin genannten Komponenten wesentlich auch für die Fig. 8 bis 11 gelten und die perspektivische Darstellung der Fig. 8 bis 11 diejenige der Fig. 4 bis 7 ergänzt.

## Patentansprüche

1. Schutzhelm, insbesondere Motorradschutzhelm, mit einer Helmschale (1), einem Kinnteil (2) zum Abdecken einer Kinnpartie eines Schutzhelmträgers, einer Kopplungsmechanik (4), durch welche das Kinnteil (2) zwischen einer unteren Position (12) und einer oberen Position (6) verschwenkbar an der Helmschale (1) befestigt ist und einem Visier (3), welches beim Kinnteil (2) in der unteren Position (12) durch die Kopplungsmechanik (4) eine jeweilige Schwenkposition zwischen einer geschlossenen Position (7) zur zumindest teilweisen Abdeckung eines Sichtbereichs des Schutzhelmträgers und einer geöffneten Position zum Freigeben des Sichtbereichs einnehmen kann, **dadurch gekennzeichnet, dass** die Kopplungsmechanik (4) dazu eingerichtet ist, auf ein Verschwenken des Kinnteils (2) aus der oberen Position (6) in die untere Position (12) nach einem vorherigen Verschwenken des Kinnteils (2) aus der unteren Position (12) in die obere Position (6) zu bewirken, dass das Visier (3) eine von der geschlossenen Position abweichende Zuvor-Schwenkposition (8) innehat, welche Zuvor-Schwenkposition (8) das Visier (3) vor dem Verschwenken des Kinnteils (2) aus der unteren Position (12) in die obere Position (6) innehatte.

2. Schutzhelm nach Anspruch 1, **dadurch gekennzeichnet, dass** die Kopplungsmechanik (4) dazu eingerichtet ist, auf ein Verschwenken des Kinnteils (2) aus der unteren Position (12) in die obere Position (6) bei dem Visier (3) in der geöffneten Position das Kinnteil (2) derart zum Visier (3) zu bewegen, dass das Visier (3) in der oberen Position (6) des Kinnteils (2) näher am Kinnteil (2) ist.

3. Schutzhelm nach Anspruch 1 oder 2, **dadurch gekennzeichnet, dass** auf ein Verschwenken des Kinnteils (2) aus der unteren Position (12) in die obere Position (6) bei einem Visier (3) in der geschlossenen Position (7) das Kinnteil (2) seine relative Lage zum Visier (3) behält, vorzugsweise, dass auf ein Verschwenken des Kinnteils (2) aus der unteren Position (12) in die obere Position (6) bei einem Visier (3) in der geschlossenen Position (7) das Visier durch das Kinnteil (2) und/oder durch die Kopplungsmechanik (4) mitgenommen wird.

4. Schutzhelm nach einem der Ansprüche 1 bis 3, **dadurch gekennzeichnet, dass** die Kopplungsmechanik (4) eine Rastvorrichtung (5) aufweist, welche schwenkbar mit der Helmschale (1) gekoppelt ist, vorzugsweise, dass das Visier (3) zum Verändern der Schwenkposition mit der Rastvorrichtung (5) schwenkbar gekoppelt ist, insbesondere, dass das Visier (3) mit der Rastvorrichtung (5) eine jeweilige Rastverbindung in einer Vielzahl von relativen Lagen zum Rastvorrichtung (5) eingehen kann.

5. Schutzhelm nach Anspruch 4, **dadurch gekennzeichnet, dass** die Rastvorrichtung (5) gegenüber dem Kinnteil (2) schwenkbar gelagert ist, insbesondere, dass die Rastvorrichtung (5) dazu eingerichtet ist, bei einem Schwenken der Rastvorrichtung (5) bezogen auf die Helmschale (1) das Visier (3) mitzunehmen, vorzugsweise durch die jeweilige Rastverbindung.

6. Schutzhelm nach Anspruch 4 oder 5, **dadurch gekennzeichnet, dass** die Kopplungsmechanik (4) eine lösbare Arretierungsvorrichtung zum starren Koppeln der Rastvorrichtung (5) mit dem Kinnteil (2) aufweist.

7. Schutzhelm nach einem der Ansprüche 1 bis 6, **dadurch gekennzeichnet, dass** die Kopplungsmechanik (4) einen, vorzugsweise starr mit dem Kinnteil (2) verbundenen, Rastmitnehmer (9) aufweist, insbesondere, dass der Rastmitnehmer (9) in der unteren Position (12) des Kinnteils (2) derart mit der Rastvorrichtung (5) in Eingriff steht, dass die Rastvorrichtung (5) in einer Schließposition (11) der Rastvorrichtung (5) fixiert ist.

8. Schutzhelm nach Anspruch 7, **dadurch gekennzeichnet, dass** die Kopplungsmechanik (4) dazu eingerichtet ist, auf eine Betätigung des Visiers (3) bei der durch den Rastmitnehmer (9) fixierten Rastvorrichtung (5) zu bewirken, dass das Visier (3) seine Schwenkposition durch eine Veränderung seiner relativen Lage zu der Rastvorrichtung (5) verändert.

9. Schutzhelm nach Anspruch 7 oder 8, **dadurch gekennzeichnet, dass** in der oberen Position (6) des Kinnteils (2) eine Schwenkbewegung der Rastvorrichtung (5) um die Helmschale (1) in mindestens eine Richtung blockierungsfrei ist.

10. Schutzhelm nach einem der Ansprüche 1 bis 9, **dadurch gekennzeichnet, dass** die Kopplungsmechanik (4) einen, vorzugsweise starr mit dem Kinnteil verbundenen, Visiermitnehmer (13) aufweist, insbesondere, dass der Visiermitnehmer (13) mit dem Visier (3) in Eingriff steht, wenn das Kinnteil (2) in der unteren Position (12) und das Visier (3) in der geschlossenen Position (7) ist.

11. Schutzhelm nach Anspruch 10, **dadurch gekennzeichnet, dass** auf ein Verschwenken des Kinnteils (2) aus der unteren Position (12) in die obere Position (6) bei dem Visier (3) in der geschlossenen Position (7) das Visier (3) durch den Visiermitnehmer (13) mitgenommen wird, vorzugsweise, dass durch das Mitnehmen des Visiers (3) durch den Visiermitnehmer (13) das Visier (3) die Rastvorrichtung (5) mitnimmt.

12. Schutzhelm nach Anspruch 10 oder 11, **dadurch gekennzeichnet, dass** der Visiermitnehmer (13) und/oder der Rastmitnehmer (9) in einem Seitenbereich des Schutzhelms angeordnet ist, vorzugsweise, dass die Kopplungsmechanik (4) beidseitig und im Wesentlichen gegenüberliegend in einem jeweiligen Seitenbereich des Schutzhelms angeordnet ist.

13. Schutzhelm nach Anspruch 11 oder 12, **dadurch gekennzeichnet, dass** in der oberen Position (6) des Kinnteils (2) eine Schwenkbewegung des Visiers (3) in Richtung der geschlossenen Position durch den Visiermitnehmer (13) und weiter in Richtung der geöffneten Position durch einen starr mit der Helmschale (1) verbundenen Oberanschlag (10) der Kopplungsmechanik (4) begrenzt wird.

14. Schutzhelm nach einem der Ansprüche 1 bis 13, **dadurch gekennzeichnet, dass** das Visier (3) und das Kinnteil (2) um eine gemeinsame Schwenkachse verschwenkt werden können, vorzugsweise, dass die Rastvorrichtung (5) um die gemeinsame Schwenkachse verschwenkt werden kann.

15. Schutzhelm nach Anspruch 14, **dadurch gekennzeichnet, dass** der Visiermitnehmer (13) einen Visiervorsprung aufweist, welcher bei einer Schwenkbewegung des Kinnteils (2) eine Schwenkbewegung in einer Schwenkebene auf einem ersten Radius um die Schwenkachse ausführt, welcher erste Radius von einem zweiten Radius um die Schwenkachse verschieden ist, auf welchem zweiten Radius die Rastvorrichtung (5) in der Schwenkebene um die Schwenkachse angeordnet ist.

## Claims

1. Protective helmet, in particular motorcycle protective helmet, having a helmet shell (1), a chin part (2) for covering a chin portion of a protective helmet wearer, a coupling mechanism (4), by means of which the chin part (2) is fastened to the helmet shell (1) so as to be pivotable between a lower position (12) and an upper position (6), and a visor (3), which, with the chin part (2) in the lower position (12), by means of the coupling mechanism (4), can assume a respective pivoting position between a closed position (7) for at least partially covering a field of view of the protective helmet wearer and an open position for freeing the field of view, **characterized in that** the coupling mechanism (4) is designed, upon pivoting of the chin part (2) from the upper position (6) to the lower position (12) after the chin part (2) has previously been pivoted from the lower position (12) to the upper position (6), to cause the visor (3) to occupy a pre-pivoting position (8) differing from the closed position, which pre-pivoting position (8) the visor (3) occupied before the pivoting of the chin part (2) from the lower position (12) to the upper position (6).

2. Protective helmet according to Claim **1, characterized in that** the coupling mechanism (4) is designed, upon pivoting of the chin part (2) from the lower position (12) to the upper position (6) with the visor (3) in the open position, to move the chin part (2) towards the visor (3) in such a way that the visor (3) is closer to the chin part (2) in the upper position (6) of the chin part (2).

3. Protective helmet according to Claim 1 or 2, **characterized in that**, upon pivoting of the chin part (2) from the lower position (12) to the upper position (6) with a visor (3) in the closed position (7), the chin part (2) maintains its position relative to the visor (3), preferably **in that**, upon pivoting of the chin part (2) from the lower position (12) to the upper position (6) with a visor (3) in the closed position (7), the visor is carried along by the chin part (2) and/or by the coupling mechanism (4).

4. Protective helmet according to one of Claims 1 to 3, **characterized in that** the coupling mechanism (4) has a latching device (5), which is pivotably coupled to the helmet shell (1), preferably **in that** the visor (3) is pivotably coupled to the latching device (5) for changing the pivoting position, in particular **in that** the visor (3) can form a respective latching connection with the latching device (5) in a plurality of positions relative to the latching device (5).

5. Protective helmet according to Claim 4, **characterized in that** the latching device (5) is pivotably mounted relative to the chin part (2), in particular **in that** the latching device (5) is designed to take along the visor (3) when the latching device (5) is pivoted relative to the helmet shell (1), preferably by means of the respective latching connection.

6. Protective helmet according to Claim 4 or 5, **characterized in that** the coupling mechanism (4) has a releasable locking device for rigid coupling of the latching device (5) with the chin part (2).

7. Protective helmet according to one of Claims 1 to 6, **characterized in that** the coupling mechanism (4) has a latch carrier (9), preferably rigidly connected to the chin part (2), in particular **in that** the latch carrier (9) engages with the latching device (5) in the lower position (12) of the chin part (2) in such a way that the latching device (5) is fixed in a closed position (11) of the latching device (5).

8. Protective helmet according to Claim 7, **characterized in that** the coupling mechanism (4) is designed to cause the visor (3) to change its pivoting position by changing its position relative to the latching device (5) in response to actuation of the visor (3) with the latching device (5) fixed by the latch carrier (9).

9. Protective helmet according to Claim 7 or 8, **characterized in that** in the upper position (6) of the chin part (2), a pivoting movement of the latching device (5) around the helmet shell (1) is unblocked in at least one direction.

10. Protective helmet according to one of Claims 1 to 9, **characterized in that** the coupling mechanism (4) has a visor carrier (13) preferably rigidly connected to the chin part, in particular **in that** the visor carrier (13) is in engagement with the visor (3) when the chin part (2) is in the lower position (12) and the visor (3) is in the closed position (7).

11. Protective helmet according to Claim 10, **characterized in that**, upon pivoting of the chin part (2) from the lower position (12) to the upper position (6) with the visor (3) in the closed position (7), the visor (3) is carried along by the visor carrier (13), preferably **in that** by the visor (3) being carried along by the visor carrier (13), the visor (3) carries along the latching device (5).

12. Protective helmet according to Claim 10 or 11, **characterized in that** the visor carrier (13) and/or the latch carrier (9) is arranged in a side region of the protective helmet, preferably **in that** the coupling mechanism (4) is arranged on both sides substantially opposite each other in a respective side region of the protective helmet.

13. Protective helmet according to Claim **11** or 12, **characterized in that** in the upper position (6) of the chin part (2), a pivoting movement of the visor (3) in the direction of the closed position is limited by the visor carrier (13) and further in the direction of the open position by an upper stop (10) of the coupling mechanism (4) rigidly connected to the helmet shell (1).

14. Protective helmet according to one of Claims 1 to 13, **characterized in that** the visor (3) and the chin part (2) can be pivoted about a common pivot axis, preferably **in that** the latching device (5) can be pivoted about the common pivot axis.

15. Protective helmet according to Claim 14, **characterized in that** the visor carrier (13) has a visor projection, which during a pivoting movement of the chin part (2), performs a pivoting movement in a pivot plane on a first radius about the pivot axis, which first radius is different from a second radius about the pivot axis, on which second radius the latching device (5) is arranged in the pivot plane about the pivot axis.

## Revendications

1. Casque de protection, en particulier casque de protection de moto, avec une coque de casque (1), une mentonnière (2) pour couvrir une partie de menton d'un porteur de casque de protection, un mécanisme d'accouplement (4) par lequel la mentonnière (2) est fixée pouvant pivoter sur la coque de casque (1) entre une position inférieure (12) et une position supérieure (6) et une visière (3), laquelle peut, avec la mentonnière (2) dans la position inférieure (12), adopter par le mécanisme d'accouplement (4) une position de pivotement respective entre une position fermée (7) pour couvrir au moins en partie une zone de vision du porteur de casque de protection et une position ouverte pour libérer la zone de vision, **caractérisé en ce que** le mécanisme d'accouplement (4) est agencé pour produire un pivotement de la mentonnière (2) de la position supérieure (6) à la position inférieure (12) après un pivotement préalable de la mentonnière (2) de la position inférieure (12) à la position supérieure (6), **en ce que** la visière (3) occupe une position de pivotement préalable (8) s'écartant de la position fermée, laquelle position de pivotement préalable (8) occupait la visière (3) avant le pivotement de la mentonnière (2) de la position inférieure (12) à la position supérieure (6).

2. Casque de protection selon la revendication **1, caractérisé en ce que** le mécanisme d'accouplement (4) est agencé pour déplacer la mentonnière (2) par rapport à la visière (3) sur un pivotement de la mentonnière (2) de la position inférieure (12) à la position supérieure (6) avec la visière (3) dans la position ouverte de telle manière que la visière (3) est plus près sur la mentonnière (2) dans la position supérieure (6) de la mentonnière (2).

3. Casque de protection selon la revendication 1 ou 2, **caractérisé en ce que** sur un pivotement de la mentonnière (2) de la position inférieure (12) à la position supérieure (6) avec une visière (3) dans la position fermée (7), la mentonnière (2) conserve sa position relative par rapport à la visière (3), de préférence, **en ce que** sur un pivotement de la mentonnière (2) de la position inférieure (12) à la position supérieure (6) avec une visière (3) dans la position fermée (7), la visière est entraînée par la mentonnière (2) et/ou par le mécanisme d'accouplement (4).

4. Casque de protection selon l'une quelconque des revendications 1 à 3, **caractérisé en ce que** le mécanisme d'accouplement (4) comporte un dispositif d'enclenchement (5), lequel est couplé pouvant pivoter avec la coque de casque (1), de préférence, **en ce que** la visière (3) est couplée pouvant pivoter avec le dispositif d'enclenchement (5) pour modifier la position de pivotement, en particulier, **en ce que** la visière (3) peut entrer dans une liaison d'enclenchement respective avec le dispositif d'enclenchement (5) dans un grand nombre de positions relatives par rapport au dispositif d'enclenchement (5).

5. Casque de protection selon la revendication 4, **caractérisé en ce que** le dispositif d'enclenchement (5) est logé pouvant pivoter par rapport à la mentonnière (2), en particulier, **en ce que** le dispositif d'enclenchement (5) est agencé pour entraîner la visière (3) lors d'un pivotement du dispositif d'enclenchement (5) par rapport à la coque de casque (1), de préférence par la liaison d'enclenchement respective.

6. Casque de protection selon la revendication 4 ou 5, **caractérisé en ce que** le mécanisme d'accouplement (4) comporte un dispositif de blocage amovible pour l'accouplement fixe du dispositif d'enclenchement (5) avec la mentonnière (2).

7. Casque de protection selon l'une quelconque des revendications 1 à 6, **caractérisé en ce que** le mécanisme d'accouplement (4) comporte un entraîneur d'enclenchement (9) relié de préférence fermement à la mentonnière (2), en particulier, **en ce que** l'entraîneur d'enclenchement (9) est dans la position inférieure (12) de la mentonnière (2) se trouve en prise avec le dispositif d'enclenchement (5) de telle manière que le dispositif d'enclenchement (5) est fixé dans une position de fermeture (11) du dispositif d'enclenchement (5).

8. Casque de protection selon la revendication 7, **caractérisé en ce que** le mécanisme d'accouplement (4) est agencé pour produire un actionnement de la visière (3) avec le dispositif d'enclenchement (5) fixé par l'entraîneur d'enclenchement (9), **en ce que** la visière (3) modifie sa position de pivotement par une modification de sa position relative par rapport au dispositif d'enclenchement (5).

9. Casque de protection selon la revendication 7 ou 8, **caractérisé en ce que** dans la position supérieure (6) de la mentonnière (2), un mouvement de pivotement du dispositif d'enclenchement (5) autour de la coque de casque (1) est sans blocage dans au moins une direction.

10. Casque de protection selon l'une quelconque des revendications 1 à 9, **caractérisé en ce que** le mécanisme d'accouplement (4) comporte un entraîneur de visière (13) relié de préférence fermement à la mentonnière, en particulier, **en ce que** l'entraîneur de visière (13) est en prise avec la visière (3), lorsque la mentonnière (2) est dans la position inférieure (12) et la visière (3) dans la position fermée (7).

11. Casque de protection selon la revendication 10, **caractérisé en ce que** sur un pivotement de la mentonnière (2) de la position inférieure (12) à la position supérieure (6) avec la visière (3) dans la position fermée (7), la visière (3) est entraînée par l'entraîneur de visière (13), de préférence, **en ce que** la visière (3) entraîne le dispositif d'enclenchement (5) par l'entraînement de la visière (3) par l'entraîneur de visière (13).

12. Casque de protection selon la revendication 10 ou 11, **caractérisé en ce que** l'entraîneur de visière (13) et/ou l'entraîneur d'enclenchement (9) est disposé dans une zone latérale du casque de protection, de préférence, **en ce que** le mécanisme d'accouplement (4) est disposé des deux côtés et pour l'essentiel à l'opposé dans une zone latérale respective du casque de protection.

13. Casque de protection selon la revendication 11 ou 12, **caractérisé en ce que** dans la position supérieure (6) de la mentonnière (2) un mouvement de pivotement de la visière (3) en direction de la position fermée est limité par l'entraîneur de visière (13) et en plus dans la direction de la position ouverte par une butée supérieure (10) du mécanisme d'accouplement (4) reliée fermement à la coque de casque (1).

14. Casque de protection selon l'une quelconque des revendications 1 à 13, **caractérisé en ce que** la visière (3) et la mentonnière (2) peuvent être pivotées autour d'un axe de pivotement commun, de préférence **en ce que** le dispositif d'enclenchement (5) peut être pivoté autour de l'axe de pivotement commun.

15. Casque de protection selon la revendication 14,
**caractérisé en ce que** l'entraîneur de visière (13) comporte une saillie de visière, laquelle exécute un mouvement de pivotement dans un plan de pivotement sur un premier rayon autour de l'axe de pivotement lors d'un mouvement de pivotement de la mentonnière (2), lequel premier rayon est différent d'un deuxième rayon autour de l'axe de pivotement, deuxième rayon sur lequel est disposé le dispositif d'enclenchement (5) dans le plan de pivotement autour de l'axe de pivotement.
